# EUROPEAN PATENT SPECIFICATION

(11) **EP 2 908 791 B1**
(45) Date of publication and mention of the grant of the patent: **05.10.2016**
(21) Application number: 13818397.5
(22) Date of filing: 17.10.2013
(51) Int. Cl.: A61F 13/02, A61K 9/70, A61M 35/00

(54) **HEATING PLASTER FOR THE TREATMENT OF PUNCTURES OF JELLYFISH AND OTHER MARINE ANIMALS**
ERWÄRMUNGSPFLASTER ZUR BEHANDLUNG VON PUNKTIONEN DURCH QUALLEN UND ANDERE MEERESTIERE
EMPLÂTRE CHAUFFANT POUR LE TRAITEMENT DES PERFORATIONS DE MÉDUSES ET D'AUTRES ANIMAUX MARINS

(30) Priority: 17.10.2012 IT FI20120211
(43) Date of publication of application: 26.08.2015
(73) Proprietor: Innovative Lab S.r.l., 56021 Cascina (Pisa) (IT)
(72) Inventor: VINCHESI, Massimo, 57023 Cecina (Livorno) (IT); FIAMINGO, Francesca Lidia, 56021 Cascina (Pisa) (IT); PONTE, Orsolina, 56040 Montescudaio (Pisa) (IT)
(74) Representative: Brazzini, Silvia
(86) International application number: PCT/IB2013/059428
(87) International publication number: WO 2014/060988

(56) References cited:
- EP-A2- 1 726 302
- WO-A2-2004/064786
- KR-A- 20030 068 625

## Description

### Field of the invention

The present invention relates in general to the pharmaceutical field, and more specifically it refers to a heating plaster for the treatment of punctures of jellyfish and other marine animals, such as scorpion fishes, rays, weevers, sea anemones and similar.

### State of the art

Jellyfishes are marine animals that are extremely widespread, present in almost all the seas of the world, although the geographical distribution of the several species of jellyfish is different, and different is also their degree of hazard.

Some species present in particular in Australia, Mexico and Southeast Asia, are extremely dangerous; deadly attacks from these species are reported every year for people, while other species, such as those located in the Mediterranean Sea, have a much lower degree of hazard. Nevertheless, even the species considered as most harmless can cause very annoying, inflammatory reactions by simple contact with the skin and, if not treated promptly in an appropriate manner, can give rise to cutaneous, more or less serious, lesions even at a distance of time.

For all species of jellyfish, such reactions on the skin are caused by contact with the stinging tentacles of the jellyfish, hanging from the lower part of the so-called umbrella-shaped bell, which constitutes the body of the animal. In the tentacles there are special cellular formations called *cnidocytes* that are activated by contact causing the extroversion of stinging filaments called *cnidae.* These filaments can be of two types, called *spirocysts* and *nematocysts,* and are connected to the so-called *cnidoblasts,* that are organelles containing an stinging liquid having a neurotoxic action. The composition of the stinging liquid may vary from species to species, but it is generally constituted by a mixture of three proteins: the hypnotoxin having an anesthetic-paralyzing effect; the thalassin, having allergenic action with inflammatory response, and the congestine, having a paralyzing effect on the circulatory and respiratory system.

More specifically, the stinging liquid released by stinging jellyfish causes an acute inflammatory reaction, characterized by erythema, swelling, blisters and bubbles, also accompanied by a burning and pain sensation. For this reason the term "burn", but also the term "sting", is often used to indicate the consequences of the contact of the jellyfish stinging liquid with the skin, as it will be in the following description of the invention. In certain cases, the immediate, acute reaction is also followed, even at a distance of days or even months from the contact with the animal, by the formation of skin lesions of eczematous type, even serious ones, or of recurrent dermatitis.

The amount of reactions related to the jellyfish sting is however in any case such as to require an effective and targeted therapeutic treatment, which, however, does not exist today. The scientific medical literature on the subject shows that there is still a big "therapeutic confusion" regarding the treatment of jellyfish stings, and any international, officially recognized, therapeutic protocol totally lacks. Many people make use of traditional remedies, void of any scientific evidence, that are ineffective, if not even harmful in certain cases; an example is the use of ammonia or urine, the use of washings with freshwater or the use of pressure bandages, all measures that may have harmful effects, resulting in the release of stinging liquid under the skin in larger amount than that released without any treatments. This is due to the fact that the filaments containing the stinging liquid, besides releasing the stinging liquid on the skin following to the contact with the animal, are also retained partially by the epidermis and, if not extracted, they may open and release *in situ* further liquid. The remedies described above just facilitate the opening of filaments, thus worsening the patient's condition.

Other remedies of the folk medicine, such as the empirical use of a heat source, such as hot surfaces and/or hot objects like rocks, engine parts or tubular inflatable boats, as well as the use of vinegar, of topical anesthetics, or of seawater have instead proved useful, at least to alleviate the pain or to prevent opening of the filaments, which are retained subcutaneously.

WO2004064786 describes an abrasive pad useful for delivering a skin treatment for marine stings, but no external layer is present as heat source.

None of the single remedies used until today, however, represent a complete and effective treatment of the consequences of jellyfish stings. To the best knowledge of the Applicant, therefore, it is still felt the need to provide an effective treatment method, which is able to act positively in a synergistic way and simultaneously against the pain, the stinging liquid already injected and the filaments retained in the epidermis.

The same observations mentioned above can also be made for other marine animals, in addition to jellyfish, such as scorpion fishes, weevers, rays, anemones and the like, that share with the jellyfish the characteristic to inoculate a type of poison constituted by thermolabile proteins and to lose filaments or stings still containing the poison, which may be retained by the epidermis. Even for the bites of these animals, the same traditional treatments have been used until today with similar results to those described above, and with the need, therefore, to develop a method of treatment finally effective against the poison injected by the animal following the puncture, as well as against stings and filaments retained by the epidermis and against the pain caused.

### Summary of the invention

The Applicant has now developed a new medical device in the form of a plaster, for use in the treatment of punctures of marine animals able to exert a synergistic and simultaneous action against the various consequences of stings from jellyfish, scorpion fishes, weevers, rays, sea anemones and the like, capable in particular of:
exerting a controlled, localized heating that inactivates the stinging liquid, decomposing the thermolabile protein components of the stinging liquid itself, which thus loses its toxic and stinging action;
retaining and incorporating any possible stings or filaments of the tentacles of the animal still containing some stinging liquid, thus preventing the release of further stinging liquid;
further releasing at least one active substance having anesthetic and/or anti-inflammatory and/or astringent and/or soothing action;
removing, during the removal of the plaster, any stings or filaments possibly incorporated.

This plaster, then, in addition to fight effectively and at the same time the various negative consequences of the punctures of the above said animals, also represents a highly practical therapeutic product, which brings together different means of treatment in a single product, which is easily transportable and always available.

It is therefore subject of the present invention a plaster for the treatment of punctures of marine animals such as jellyfish, scorpion fishes, weevers, rays, sea anemones and the like, comprising at least two layers, of which a first layer intended for contacting the skin is capable of retaining, incorporating and removing stings or stinging filaments of the animal possibly present in the epidermis, and a second external layer has heating action activated at time of use.

A kit comprising the present plaster and a device suitable for the removal of stings or filaments, as well as the use of the present plaster or of the kit, represent further subjects of the invention.

Further important features of the product of the invention are disclosed in the following detailed description.

### Detailed description of the invention

In the present invention, the expression "puncture of animals" means the contact of the stinging animal with a body's epidermis, with consequences that require a therapeutic treatment.

The first layer of the present plaster, intended for contacting the skin, comprises a viscous matrix, consisting essentially of products in form of waxes or gels which are stable at least up to a temperature of approximately 60°C, selected from amongst polysulphones, polycarbonates, polyurethanes, alginates, and synthetic polymers such as polymethacrylates, polysiloxanes and the like. Typically, this viscous matrix is constituted by a wax or a gel which take a highly viscous consistency following heating, when the outer heating layer activates, while they decrease their viscosity up to solidify when the plaster cools down to body temperature; in this way, during the heating phase, the matrix easily incorporates the filaments that may be present on the epidermis, holding them in the matrix solidified during removal of the patch.

By the term "body temperature" according to the present invention, a normal temperature of a healthy human body is meant, comprised typically between 36°C and 38°C.

According to a preferred embodiment of the invention, the layer of the present plaster intended for contacting the skin further comprises one or more active principles having deactivating action of the stinging liquid already injected in the epidermis, and having anti-inflammatory, anaesthetic, astringent and/or soothing action, optionally in combination with agents that improve the active principles' release and passage through the epidermis, and/or with other excipients and/or diluents that are pharmaceutically acceptable and commonly used in this kind of formulations; such active principles are preferably selected from the group consisting of acetic acid, aloe, allantoin, aluminium chloride, lidocaine, benzocaine, cortisonics, thermostable proteolytic enzymes, such as bromelain or papain, calcium or lanthanum salts, grapefruit seed extracts, and Tea Tree oil. These active principles may be formulated in a "traditional" way or in such a way as to obtain a modified, delayed or controlled release, for instance by formulating the active principle in the form of microcapsules or nanoparticles or a similar formulation suitable for modifying the release of the same active principle in the desired way.

The inner layer of the present plaster, besides having a therapeutic action when are present one or more active principles for the local transdermal release, has mainly a "cushion" action, maintaining intact the stinging filaments of the jellyfish or the stings of other animals that may be present in the area to be treated, which remain embedded and protected within a matrix that has high viscosity and constitutes this layer of the plaster. In this way, the breaking of the filaments themselves is avoided, as well as the consequent release of further stinging liquid that would worsen the consequences of the puncture. This effect is also promoted by the presence of sea water on the epidermis' area to be treated on which the plaster is applied; as a matter of fact, the viscous layer helps retaining on this area sea water, whose saline concentration is known to be useful to prevent opening of the stinging filaments, which are possibly retained subcutaneously.

Furthermore, the particular nature of the matrix, having a viscosity variable with temperature, causes the filaments that may be present on the epidermis to be retained inside the matrix when removing the plaster. It is a mechanical action whereby the filaments, still intact, remain adherent to the viscous matrix when the plaster is removed, avoiding the release of the stinging liquid contained in the filaments.

This inner layer of the plaster finally performs an adhesive action, making the plaster adhere to the skin, even *per se* thanks to the high viscosity matrix, without the need for adhesive edges.

Alternatively, the present plaster may be further provided with two or more adhesive edges for the adhesion of the plaster to the skin, preferably made with adhesive products such as polysiloxanes, polyacrylates, ethylene-vinyl acetate copolymers, polyurethanes and elastomeric polymers such as polyisobutene. Particularly suitable to the invention are silicone derivatives in reticular or linear form that well tolerate high temperatures. In general, products that do not create irritation to the skin are preferred.

The present plaster further comprises an outer heating layer that, at time of use, is activated thus generating a heating up to a maximum temperature of approximately 60°C, preferably up to a temperature ranging between 40 and 50°C, more preferably between 42 and 48°C, for a period of time ranging for instance between 20 and 30 minutes, and preferably shorter than 30 minutes. The Applicant has found in particular that the heating action of the plaster on the lesion from jellyfish puncture in such conditions of times and temperature, is able to inactivate the thermolabile proteins present in the stinging liquid injected in the lesion, thus having a first desired therapeutic effect.

The outer heating layer can be prepared in such a way as to be easily detached from the other layers of the plaster once spent the time of 20-30 minutes considered necessary and sufficient to exert the therapeutic action of the heating, in case the plaster has to be maintained on the skin, for instance because it contains active ingredients in the inner layer that may also act advantageously for longer times.

The heating layer according to the invention can be prepared for example with a supersaturated aqueous solution of sodium acetate and a sheet of metal of suitable dimensions, inserted inside a casing of plastic material resistant to heat and preferably also capable of catalyzing the exothermic reaction occurring inside it, further distributing the heat over the entire surface of the plaster. The supersaturated aqueous solution of sodium acetate can be prepared starting from an aqueous solution obtained by heating at about 100°C sodium acetate trihydrate in the crystalline form, and subsequent cooling to room temperature. Within the above said casing, when the metal sheet is pressed, a nucleation center forms that causes the crystallization of sodium acetate, which takes the trihydrate crystalline form; this reaction is exothermic and releases the amount of heat needed for the purpose required for the therapeutic plaster of the invention.

The heating action of the outer layer can also be provided by two separate housings comprised in the outer layer, respectively comprising water or other suitable liquid and a reagent that, by contact with the liquid, generates heat at the temperature and for the time desired, for instance comprising water and calcium chloride.

Alternatively, the heating layer can be made from a formulation which, by contact with oxygen in the air, gives rise to a highly exothermic oxidation reaction, thus producing enough heat to raise the temperature to the above said values for the time necessary for the treatment; for instance, such a formulation comprises iron powder, iron sulphate or carbonate, vermiculite, sodium chloride or the like, optionally in the presence of a suitable catalyst of the oxidation reaction. An external protective film capable of preventing the passage of air will also be comprised in the plaster, so that the heating layer activates only as a result of the opening of the film, and the passage of oxygen is prevented until use. In this case the plaster is manufactured and packaged under protected atmosphere, and then sealed in a special casing impermeable to gases, so as to guarantee the absence of air until use.

According to a further embodiment of the invention, the outer layer comprises electrical microresistances able to produce heat by means of electricity supplied from microbatteries or by connection to the electricity distribution network, inserted inside a plastic heat-resistant casing. In this embodiment, the present plaster may provide for the union of the two layers, the external heating layer and the inner high viscosity layer, in a single layer in which the electrical microresistances are embedded in a matrix of a viscous heat-resistant polymer, wherein are also possibly present also one or more active ingredients as described above, so that they may also be released through electroporation.

According to a particular embodiment of the invention, the present plaster may also comprise a further layer adjacent to the heating layer or to the support layer, if present; this additional layer, called cooling layer, has just the function of cooling the surface of the plaster, and therefore the area to be treated, thanks to appropriate liquids or crystals having thermal action and activated by the rupture of the casing in which they are enclosed. The activation of the two opposite thermal actions, the cooling and heating actions, takes place sequentially, since the heating action is suitable to inactivate the poison prior to the cooling action, which has astringent, anti-edema and anti-inflammatory function useful to treat the epidermis after inactivation of the poison thanks to the heat.

According to a particular embodiment of the present plaster, an additional layer is present between the external heating layer and the inner viscous layer, such additional layer having function of support and/or protection of the skin from a possible excessive heat generated by the outer heating layer, and possibly also a function of adhesive to hold the plaster in position on the area of skin to be treated.

The dimensions of the present plaster may be the most different, so as to adapt it to the various dimensions of lesions, and the thickness is preferably limited in order to facilitate the application of the plaster on the skin. The plaster of the invention may have different shapes, such as rectangular, square, ribbon-like, and at least two of its edges may for instance be free from the internal matrix with high viscosity, or this matrix fills the entire surface available of the inner layer. The square or rectangular shapes with right angles are preferred, because they allow the continued application of multiple plasters in adjacent areas, also covering very large areas of the body, without leaving any spots uncovered and without the therapeutic action of the plaster.

In any case, the plaster of the invention is preferably sealed inside a suitable protective container until use; this container is made of a material impermeable to substances present on the plaster, adhesive but easily detachable from the plaster.

A preferred embodiment of the present plaster provides for the use of only transparent materials, so as to maintain, at all times of the treatment, a visual contact also with the lesion, in order to evaluate the progress in real time.

According to a particular embodiment of the present plaster, it may comprise a temperature indicator, for example an LCD (Liquid Crystals Display) indicator, to facilitate control of the heat generated and to further improve the use of the plaster.

As an example, a plaster according to the present invention may be prepared by using a viscous matrix consisting essentially of calcium and sodium alginate, and polymethacrylate, which is spread on a surface of a polyester support layer, thus forming a uniform viscous layer intended for contacting the skin. The opposite surface of this support layer is attached to the external heating layer, that may be prepared with a mixture of iron powder, vermiculite, sodium chloride and active carbon as the catalyst, put inside a plastic film permeable to air. This operation is carried out under inert atmosphere, as well as the operation of packaging the so-obtained plaster device in an external protective film made of polypropylene, which prevents the passage of air until use and may be easily broken at time of use to allow air flowing inside the heating layer and trigger the heating action.

The present plaster, as described above, may be packaged in a kit together with a plastic device, for example in the shape of tweezers or spatula, suitable for the removal of that part of the stings or stinging filaments retained on the surface and more easily visible, before application of the plaster.

In conclusion, the present plaster for the therapeutic treatment of lesions from contact with jellyfish and other marine animals mentioned above acts in a synergistic way and simultaneously against the various consequences of the contact with the skin, inactivating the injected stinging liquid, composed of thermolabile proteins, thanks to a thermal action, blocking the opening of further filaments or stings of the animal that may be retained on the epidermis and removing them still intact when removing the plaster. The optional presence of active ingredients in the inner adhesive layer allows conveying anesthetics, anti-inflammatory, astringent and/or soothing agents on the injured area, with an effective therapy and prevention of damage from burns caused by contact with the animal. Due to its formulation as a plaster, it is a therapeutic mean particularly handy and easy to carry, even in places far from first-aid posts, and it is also easy to use.

## Claims

1. A plaster for use in the treatment of punctures of marine animals such as jellyfishes, scorpion fishes, weevers, rays, sea anemones and similar, on a body's epidermis comprising at least two layers, of which a first layer intended to contact the skin to be treated is suitable for holding, incorporating and removing stings and stinging filaments of said animals possibly present in the epidermis following said punctures and comprises a matrix consisting essentially of waxes or products which becomes highly viscous by heating at a temperature higher than the body temperature, and then solidify by cooling at the body temperature or at lower temperature, and a second more external layer has a heating action that is activated at time of use for a period of time shorter than 30 minutes.

2. The plaster for use according to claim 1, wherein said products are gels stable at temperature up to at least 6(3°C selected from the group consisting of polysulfones, polycarbonates, polyurethanes, aiginates, and synthetic polymers such as polymethacrylates, polysiloxanes and similar.

3. The plaster for use according to claims 1-2, further comprising one or more active principles having deactivating action towards a stinging liquid possibly injected by said animals in epidermis, and/or anti-inflammatory, anaesthetic, astringent and/or soothing action.

4. The plaster for use according to claim 3, further comprising one or more agents improving delivery and penetration through epidermis of said active principles and/or further pharmaceutically acceptable excipients or diluents.

5. The plaster for use according to claim 3, wherein said active principles are selected from the group consisting of acetic acid, aloe, allantoin, aluminium chloride, lidocaine, benzocaine, cortisonics, thermostable proteolytic enzymes, such as bromelain and papain, calcium or lanthanum salts, grapefruit seeds extracts, and Tea Tree oil.

6. The plaster for use according to claim 1, wherein said second external layer is activated generating heating to a temperature ranging from 40 and 50°C for a time comprised between 20 and 30 minutes.

7. The plaster for use according to claim 6, wherein said temperature ranges between 42 and 48°C.

8. The plaster for use according to claims 1-7, wherein said second layer may be removed thus leaving in position on the skin the remaining layers of the plaster.

9. The plaster for use according to claims 1-8, further comprising at least a further intermediate layer between said external heating second layer and said internal viscous first layer, said intermediate layer having function of support and/or protection for the skin from any possible excess of heat generated from said external layer.

10. The plaster for use according to claims 1-9, further comprising a further layer adjacent to said intermediate layer, if present, or to said heating layer, said further layer having cooling, antiedema, astringent and/or anti-inflammatory function, that may be activated in sequence once the heating action is finished.

11. The plaster for use according to claims 1-10, further comprising a temperature indicator.

12. The plaster for use according to claim 1, wherein said first and second layer are joined in a single layer comprising electric micro-resistances incorporated in a matrix of a suitable thermoresistant viscous polymer, possibly further comprising one or more active principle having a deactivating action towards the stinging liquid already injected by said marine animal in epidermis, and/or having anti-inflammatory, anaesthetic, astringent and/or soothing action.

13. A kit for use in the treatment of punctures of marine animals such as jellyfishes, scorpion fishes, weevers, rays, sea anemones and similar, comprising the plaster for use as described in claims 1-12 and a device suitable for removing stings or stinging filaments of said animals possibly present in epidermis.

## Patentansprüche

1. Pflaster zur Verwendung bei der Behandlung vom Punktionen von Meerestieren, wie Quallen, Skorpionfischen, Petermännchen, Rochen, Seeanemonen und ähnliches, auf einer Epidermis eines Körpers, wobei wenigstens zwei Schichten enthalten sind, von denen eine erste Schicht, die vorgesehen ist, um mit der Haut in Kontakt zu kommen, die zu behandeln ist, geeignet ist zum Halten, Beinhalten und Entfernen von Stacheln und stacheligen Filamenten der Tiere, die möglicherweise in der Epidermis auf die Punktionen folgend vorhanden sind, und eine Matrix enthält, die im Wesentlichen aus Wachsen oder Produkten besteht, die durch Erwärmen auf eine Temperatur, die höher als die Körpertemperatur ist, hoch viskos werden und sich dann durch Abkühlen auf die Körpertemperatur oder eine niedrigere Temperatur verfestigen, und eine zweite, mehr außen liegende Schicht eine Erwärmungswirkung hat, die zum Zeitpunkt der Anwendung für eine Zeitperiode aktiviert wird, die kürzer als 30 Minuten ist.

2. Pflaster zur Verwendung nach Anspruch 1, wobei die Produkte bei einer Temperatur bis hin zu 60 ° C stabile Gele sind, die aus der Gruppe ausgewählt sind, die aus Polysulfonen, Polycarbonaten, Polyurethanen, Alginaten, und sysnthetischen Polymeren, wie Polymethacrylaten, Polysiloxanen und ähnlichem, besteht.

3. Pflaster zur Verwendung nach einem der Ansprüche 1 bis 2, wobei ferner ein aktives Prinzip oder mehrere aktive Prinzipien enthalten ist/sind, das/die eine deaktivierende Wirkung hinsichtlich einer stechenden Flüssigkeit, die möglicherweise durch die Tiere in die Epidermis injiziert wurde, oder eine entzündungshemmende, betäubende, astringente und/oder beruhigende Wirkung hat/haben.

4. Pflaster zur Verwendung nach Anspruch 3, wobei ferner ein oder mehrere Mittel enthalten ist/sind, das/die die Zufuhr oder Penetration der aktiven Prinzipien und/oder weiterer pharmazeutisch akzeptabler Hilfsstoffe oder Verdünnungsmittel durch die Epidermis verbessert/verbessern.

5. Pflaster zur Verwendung nach Anspruch 3, wobei die aktiven Prinzipien ausgewählt sind aus der Gruppe, die aus Essigsäure, Aloe, Allantoin, Aluminiumchlorid, Lidocain, Benzocain, Kortisonen, thermostabilen proteolytischen Enzymen, wie Bromelain und Papain, Kalzium- oder Lanthansalzen, Grapefruitsamenextrakten und Teebaumöl besteht.

6. Pflaster zur Verwendung nach Anspruch 1, wobei die zweite äußere Schicht zum Erzeugen einer Erwärmung auf eine Temperatur, die von 40 bis 50 °C reicht, über eine Zeit aktiviert wird, die zwischen 20 und 30 Minuten enthalten ist.

7. Pflaster zur Verwendung nach Anspruch 6, wobei die Temperatur im Bereich zwischen 42 und 48 °C liegt.

8. Pflaster zur Verwendung nach einem der Ansprüche 1 bis 7, wobei die zweite Schicht entfernt werden kann, wodurch die verbleibenden Schichten des Pflasters auf der Haut in Position belassen werden.

9. Pflaster zur Verwendung nach einem der Ansprüche 1 bis 8, wobei wenigstens eine Zwischenschicht zwischen der äußeren erwärmenden zweiten Schicht und der inneren klebrigen ersten Schicht enthalten ist, wobei die Zwischenschicht die Funktion einer Unterstützung und/oder eines Schutzes für die Haupt vor einem möglichen Übermaß an Wärme hat, die von der äußeren Schicht erzeugt wird.

10. Pflaster zur Verwendung nach einem der Ansprüche 1 bis 9, wobei ferner eine weitere Schicht benachbart der Zwischenschicht, falls diese vorhanden ist, oder der erwärmenden Schicht enthalten ist, wobei die weitere Schicht eine kühlende, antiödeme, astringente und/oder entzündungshemmende Funktion hat, die aktiviert werden kann, sobald die wärmende Wirkung beendet ist.

11. Pflaster zur Verwendung nach einem der Ansprüche 1 bis 10, wobei ferner ein Temperaturindikator enthalten ist.

12. Pflaster zur Verwendung nach Anspruch 1, wobei die erste und die zweite Schicht in einer einzelnen Schicht verbunden sind, die elektrische Mikrowiderstände enthält, die in einer Matrix aus einem geeigneten wärmebeständigen klebrigen Polymer beinhaltet sind, wobei möglicherweise ferner ein aktives Prinzip oder mehrere aktive Prinzipien enthalten ist/sind, das/die eine deaktivierende Wirkung hinsichtlich der stechenden Flüssigkeit hat/haben, das durch das Meerestier bereits in die Epidermis injiziert wurde, und/oder eine entzündungshemmende, betäubende, astringente und/oder beruhigende Wirkung hat/haben.

13. Kit zur Verwendung bei der Behandlung vom Punktionen von Meerestieren, wie Quallen, Skorpionfischen, Petermännchen, Rochen, Seeanemonen und ähnliches, wobei das Pflaster zur Verwendung, wie in den Ansprüchen 1 bis 12 beschrieben ist, und eine Vorrichtung enthalten sind, die zum Entfernen von Stacheln und stacheligen Filamenten der Tiere geeignet sind, die möglicherweise in der Epidermis vorhanden sind.

## Revendications

1. Un emplâtre pour utilisation dans le traitement des piqûres d'animaux marins tels que les méduses, les poissons scorpions, les vives, les raies, les anémones de mer et similaires, sur l'épiderme d'un corps, comprenant au moins deux couches, dont une première couche destinée à être en contact avec la peau à traiter est adaptée pour retenir, englober et éliminer les dards et les filaments urticants des dits animaux éventuellement présents dans l'épiderme à la suite des dites piqûres et comprend une matrice constituée essentiellement de cires ou de produits qui deviennent hautement visqueux par chauffage à une température supérieure à la température du corps, et ensuite se solidifient par refroidissement à la température du corps ou à une température inférieure, et une seconde couche plus extérieure a une action de chauffage qui est activée au moment de l'utilisation pendant une période de temps inférieure à 30 minutes.

2. L'emplâtre pour utilisation selon la revendication 1, dans lequel lesdits produits sont des gels stables à une température jusqu'à au moins 60°C choisis dans le groupe constitué de polysulfones, de polycarbonates, de polyuréthanes, d'alginates, et de polymères synthétiques tels que des polyméthacrylates, des polysiloxanes et similaires.

3. L'emplâtre pour utilisation selon les revendications 1-2, comprenant en outre un ou plusieurs principes actifs ayant une action de désactivation à l'égard d'un liquide urticant éventuellement injecté par lesdits animaux dans l'épiderme et/ou une action anti-inflammatoire, anesthésique, astringente et/ou apaisante.

4. L'emplâtre pour utilisation selon la revendication 3, comprenant en outre un ou plusieurs agents améliorant la délivrance et la pénétration à travers l'épiderme des dits principes actifs et/ou d'autres excipients pharmaceutiquement acceptables ou diluants.

5. L'emplâtre pour utilisation selon la revendication 3, dans lequel lesdits principes actifs sont choisis dans le groupe constitué de l'acide acétique, l'aloès, l'allantoïne, le chlorure d'aluminium, la lidocaïne, la benzocaïne, les cortisoniques, les enzymes protéolytiques thermostables, tels que la bromélaïne et la papaïne, des sels de calcium ou de lanthane, des extraits de graines de pamplemousse, et l'huile d'arbre à thé.

6. L'emplâtre pour utilisation selon la revendication 1, dans lequel ladite seconde couche extérieure est activée pour produire un chauffage jusqu'à une température comprise entre 40 et 50°C pendant une durée comprise entre 20 et 30 minutes.

7. L'emplâtre pour utilisation selon la revendication 6, dans lequel ladite température est comprise entre 42 et 48°C.

8. L'emplâtre pour utilisation selon les revendications 1-7, dans lequel ladite seconde couche peut être retirée, en laissant ainsi en place, sur la peau, les couches restantes de l'emplâtre.

9. L'emplâtre pour utilisation selon les revendications 1-8, comprenant en outre au moins une autre couche intermédiaire entre ladite seconde couche extérieure de chauffage et ladite première couche visqueuse intérieure, ladite couche intermédiaire ayant une fonction de support et/ou de protection de la peau contre tout excès éventuel de chaleur générée par ladite couche extérieure.

10. L'emplâtre pour utilisation selon les revendications 1-9, comprenant en outre une autre couche adjacente à ladite couche intermédiaire, si elle est présente, ou à ladite couche de chauffage, ladite autre couche ayant une fonction de refroidissement, d'anti-oedème, astringente et/ou anti-inflammatoire, qui peut être activée en séquence après que l'action de chauffage ait été terminée.

11. L'emplâtre pour utilisation selon les revendications 1-10, comprenant en outre un indicateur de température.

12. L'emplâtre pour utilisation selon la revendication 1, dans lequel lesdites première et seconde couches sont réunies en une seule couche comprenant des micro-résistances électriques incorporées dans une matrice d'un polymère visqueux thermorésistant approprié, comprenant en outre le cas échéant un ou plusieurs principes actifs ayant une action de désactivation à l'égard du liquide urticant ayant été injecté par ledit animal marin dans l'épiderme, et/ou ayant une action anti-inflammatoire, anesthésique, astringente et/ou apaisante.

13. Un kit pour utilisation dans le traitement de piqûres d'animaux marins tels que les méduses, les poissons scorpions, les vives, les raies, les anémones de mer et similaires, comprenant l'emplâtre pour utilisation comme décrit dans les revendications 1-12 et un dispositif approprié pour éliminer les dards ou les filaments urticants des dits animaux éventuellement présents dans l'épiderme.
